(19) Europäisches Patentamt
European Patent Office
Office européen des brevets

(11) **EP 4 682 242 A1**
## (12) EUROPEAN PATENT APPLICATION
published in accordance with Art. 153(4) EPC

(43) Date of publication:
**21.01.2026 Bulletin 2026/04**

(21) Application number: **24770967.8**

(22) Date of filing: **14.03.2024**

(51) International Patent Classification (IPC):
*C12N 1/04* (2006.01)    *A61K 9/107* (2006.01)
*A61K 35/17* (2025.01)   *A61K 47/12* (2006.01)
*A61K 47/20* (2006.01)   *A61K 47/26* (2006.01)
*A61K 47/42* (2017.01)   *A61P 35/00* (2006.01)
*C12N 5/0783* (2010.01)

(52) Cooperative Patent Classification (CPC):
**A61K 9/107; A61K 35/17; A61K 47/12;
A61K 47/20; A61K 47/26; A61K 47/42;
A61P 35/00; C12N 1/04; C12N 5/06**

(86) International application number:
**PCT/JP2024/009978**

(87) International publication number:
**WO 2024/190864 (19.09.2024 Gazette 2024/38)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **15.03.2023  JP 2023041279**

(71) Applicant: **GAIA BioMedicine Inc.
Fukuoka 810-0041 (JP)**

(72) Inventors:
• **HARADA Yui
Fukuoka-shi, Fukuoka 819-0395 (JP)**
• **YONEMITSU Yoshikazu
Fukuoka-shi, Fukuoka 819-0395 (JP)**

(74) Representative: **Vossius & Partner
Patentanwälte Rechtsanwälte mbB
Siebertstrasse 3
81675 München (DE)**

## (54) SOLUTION FOR SUSPENDING CELLS, AND USE OF SAME

(57)    An object of the present invention is to provide a solution for thawing that can maintain or improve the cytotoxic activity of highly active NK cells and the like after thawing. A solution for suspending cells for administration to humans, which satisfies the following criteria: (1) containing potassium ion, (2) having a pH of 4.9 or higher, (3) not containing chloride ions at a concentration of 135 mEq/L or higher, (4) not containing glucose at a concentration of 5.55 mM or higher, (5) not containing calcium ions at a concentration of 0.423 mM or higher, (6) having an osmolarity of 200 to 396 mOsm, and (7) containing any organic acid(s) selected from the group consisting of succinic acid, malic acid, lactic acid, citric acid, and acetic acid.

**EP 4 682 242 A1**

## Description

Technical Field

The present invention relates to a method for treating cells for administration to humans, such as highly active NK cells.

NK cells are important for killing tumor cells and virus-infected cells. In August 2017, chimeric antigen receptor (CAR) T-cell therapy was approved in the United States for the treatment of relapsed and refractory B-cell acute lymphoblastic leukemia (B-ALL) in children and young adults, and in recent years, clinical application of CAR-NK has been expanding in place of CAR-T (Non-patent document 1).

When attempting to administer cells to a patient, the first consideration is to use cells harvested from the patient himself/herself to avoid rejection. However, depending on the patient's condition, it may be difficult to collect the necessary amount of cells for treatment. In addition, the degree to which cells can be activated and proliferated in vitro varies among individuals, and there are also cases in which proliferation and activation of cells are difficult. In addition, it takes a certain period of time for cells to be activated and proliferated, posing a problem that it is difficult to start treatment immediately. In this respect, it would be desirable if cells could be activated in advance and stored for preparation of administration.

As a method for preserving cells, it is known to preserve cells in suspension without freezing for short-time preservation (e.g., Patent document 1), and to freeze cells for long time preservation (e.g., Patent document 2). Furthermore, use of a solution for freezing containing a sodium salt, potassium salt, sugar, cryoprotectant, and hydrogencarbonate and/or carbonate has been investigated, since use of such a solution may provide cells that maintain high viability even after freeze-thawing (Patent document 3).

Also known is a method for preserving cells that are difficult to preserve, such as NK cells, in which the cells are stored in a solution containing a sodium salt, potassium salt, sugar, and protein as active ingredients (Patent document 4). Further, a cell preservation solution for refrigerated preservation containing potassium ions and lactate ions, and having an osmolarity of 200 to 350 mOsm/kg and pH of 6.0 to 8.0 is known (Patent document 5), and this preservation solution contains 0.5 to 150 mM of sugars and 1 to 4 mM of calcium ions. Furthermore, there is also known a cell or tissue preservation solution having an osmolarity of 270 to 450 mOsm/l and pH of 7 to 8, and containing $K^+$ and organic acid anions (Patent document 6). In this reference, lactic acid is exemplified as the organic acid. Furthermore, it is known to use a physiological aqueous solution as a solution for suspending mammalian cells such as NK cells, and it is known that isotonic (250 to 380 mOsm/l) aqueous solutions such as Ringer's solution (lactated Ringer's solution) can be used as the physiological aqueous solution (Patent document 7). In an example of this reference, a lactated Ringer's solution ("Lactec Injection" produced by Otsuka Pharmaceutical Factory) is used (paragraph 0038), which contains potassium and lactic acid, has a pH of 6.0 to 7.5, and does not contain glucose.

The inventors of the present invention have developed a method for treating highly active NK cells for freezing them and a solution containing sodium chloride, sodium gluconate, sodium acetate, potassium chloride, and magnesium chloride as a solution for use in unfreezing frozen highly active NK cells (Patent document 8).

Prior Art References

Patent documents

Patent document 1: Japanese Patent Publication (Kokai) No. 2006-230396
Patent document 2: Japanese Patent Publication (Kokai) No. 2002-233356
Patent document 3: WO2011/021618
Patent document 4: WO2013/115322
Patent document 5: Japanese Patent No.4947948
Patent document 6: WO2002/001952
Patent document 7: Japanese Patent Publication (Kokai) No. 2013-233102
Patent document 8: WO2021/177279
Patent document 9: PCT/JP2022/033487

Non-patent document

Non-patent document 1: Liu E, et al. N Engl J Med. 2020;382:545-53

Summary of the Invention

Object to be achieved by the invention

It would be desirable to have a solution for thawing containing more general-purpose ingredients that can maintain or improve the cytotoxic activity of highly active NK cells after thawing. It would also be desirable if such a solution is also suitable as an infusion.

Means for achieving the object

The present invention provides the followings.

[1] A solution for suspending cells for administration to humans, which satisfies the following:

(1) contains potassium ions;
(2) has a pH of 4.9 or higher;
(3) does not contain chloride ions at a concentration of 135 mEq/L or higher;
(4) does not contain glucose at a concentration of 5.55 mM or higher;
(5) does not contain calcium ions at a concentration of 0.423 mM or higher;
(6) has an osmotic pressure of 200 to 396 mOsm; and
(7) contains any organic acid(s) selected from the group consisting of succinic acid, malic acid, lactic acid, citric acid, and acetic acid.

[2] The solution according to 1, wherein the organic acid (7) is succinic acid.
[3] The solution according to 1 or 2, which further satisfies (8) containing L-cysteine or acetylcysteine.
[4] The solution according to any one of 1 to 3, which further satisfies (9) containing albumin.
[5] The solution according to any one of 1 to 4, which is for diluting a frozen product containing cells for administration to humans or a thawed product thereof.
[6] The solution according to any one of 1 to 4, which satisfies

(1) contains potassium ions at a concentration of 4.00 mEq/L or higher;
(4) does not contain glucose; and
(5) does not contain calcium ions.

[7] The solution according to any one of 1 to 6, which is a pharmaceutical composition.
[8] A pharmaceutical composition containing the solution according to any one of 1 to 4 and a population of cells for administration to humans, wherein the cells for administration to humans are lymphocytes.
[9] A pharmaceutical composition containing a population of cells for administration to 1humans suspended in the solution according to any one of 1 to 7, wherein the cells for administration to humans are highly active, NK cell-like allogeneic CD3-negative cells.
[10] The pharmaceutical composition according to 9, wherein the population of highly active, NK cell-like allogeneic CD3-negative cells has undergone a freezing process.
[11] A method for providing a pharmaceutical composition for infusion containing cells for administration to a human, which comprises the step of thawing cryopreserved cells and suspending the thawed cells in the solution according to any one of 1 to 7 to prepare a pharmaceutical composition for infusion.

Effect of the Invention

According to the present invention, the cytotoxic activity of frozen and thawed highly active NK cells etc.
As a secondary effect, by using a specific organic acid in the solution for thawing, the high cytotoxic activity of highly active NK cells can be maintained or improved even in a low pH range. Considering the ranges of specifications of existing infusion solutions, such usability in a low pH range may lead to a wider range of clinical usefulness.

Brief Description of the Drawings

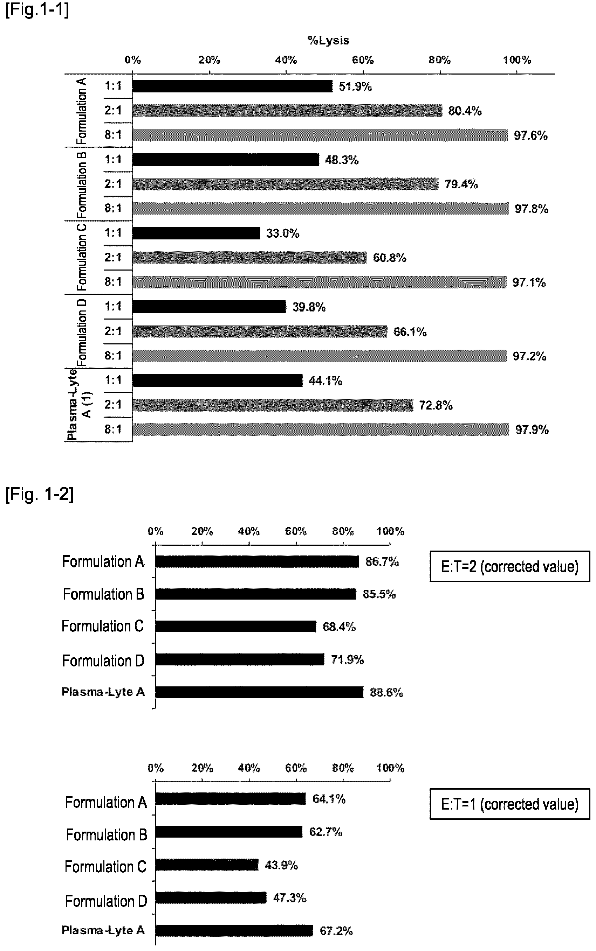

[Fig. 1-1] Cytotoxic activity against tumor cells (Example 1).

[Fig. 1-2] Cytotoxic activity against tumor cells (corrected values, Example 1).

[Fig. 2] Evaluation of pH adjusters (malic acid and succinic acid, Example 2).

[Fig. 3] Evaluation of pH adjusters (citric acid, lactic acid, and acetic acid, Example 3).

[Fig. 4-1] Evaluation of pH adjusters (ascorbic acid, Example 4).

[Fig. 4-2] Evaluation of pH adjusters (immediately after thawing, Example 4).

[Fig. 4-3] Evaluation of pH adjusters (at 4 hours after thawing and dilution, Example 4).

[Fig. 5-1] Activity after thawing (succinic acid, pH 5.9 to 8.0, Example 5).

[Fig. 5-2] Activity after thawing (succinic acid, pH 5.9 to 8.0, Example 5).

[Fig. 6-1] Activity after thawing (succinic acid, pH 4.9 to 5.9, Example 6).

[Fig. 6-2] Activity after thawing (succinic acid, pH 4.9 to 5.9, Example 6).

[Fig. 7] Summary of Examples 5 and 6. Relative % Lysis (E:T=1, corrected values) and 7-AAD-4 gate (%). The values obtained with Plasma-Lyte A are defined as 1.0. Bars indicate Mean+S.D.

Modes for Carrying out the Invention

In the explanations of the present invention, mM is used with the same meaning as mmol/L, unless especially noted. When a numerical range is expressed as x to y, the range includes the values x and y at both ends.

The present invention relates to a method for thawing frozen cells for administration to humans. For the present invention, the term thawing is used to mean thawing a frozen material, unless especially noted. Thawing may involve adding a solution to the frozen material to dilute it. Making a frozen solid into a liquid may also be referred to as unfreezing.

[Applicable cells]

The present invention can be applied to a variety of cells. One class of the cells to which the present invention can be preferably applied are cells to be administered to humans, preferably cells for administration to humans that have undergone an activation operation ex vivo, such as NK cells with high cytotoxic activity (highly active NK cells). Cells to which the present invention can be preferably applied also include GAIA-102 (highly active, NK cell-like allogeneic CD3-negative cells produced from peripheral blood mononuclear cells using amplification and activation culture techniques). Other examples of cells to which the present invention can be preferably applied include CAR-T and CAR-NK cells. The activation operation is typically based on incubating the cells with a medium containing interleukin (IL)-2. In the following descriptions, the present invention may be explained by exemplifying cases of applying it to NK cells or highly active NK cells, but those skilled in the art can also appropriately understand the application of the present invention to other types of cells on the basis of such explanations.

In general, NK cells are large granular lymphocytes that are not expressing the T cell receptor (TCR), the universal T cell marker, CD3, and the membrane immunoglobulin, B cell receptor, and they are usually CD16-positive and CD56-positive in humans. Whether or not cells are NK cells can be easily determined by those skilled in the art on the basis of expression patterns of cell surface markers, and so forth. NK cells have cytotoxic activity, and the presence or absence and degree of cytotoxic activity can be measured by various known methods. NK cells can include peripheral blood NK cells, cord blood NK cells, primary NK cells, cultured NK cells, and highly active NK cells.

(Raw material)

Raw material of highly active NK cells, highly active NK cell-like allogeneic CD3-negative cells, and so forth to which the present invention can be preferably applied may be peripheral blood, cord blood, bone marrow and/or lymph nodes, and blood collected by apheresis method (apheresis blood). The raw material may also be prepared from at least one kind of cells selected from the group consisting of hematopoietic stem cells derived from any stem cells selected from the group consisting of embryonic stem cells, somatic stem cells, and induced pluripotent stem (iPS) cells, hematopoietic stem cells derived from cord blood, hematopoietic stem cells derived from peripheral blood, hematopoietic stem cells derived from bone marrow blood, cord blood mononuclear cells, and peripheral blood mononuclear cells. The donor of the raw material may be a patient himself/herself who will receive an immunotherapy using highly active NK cells or the like, a close relative of the patient, or a healthy person genetically unrelated to the patient. The donor may consist of a plurality of donors.

(Culture medium)

Examples of the culture medium used for culturing highly active NK cells and so forth include the KBM501 medium (Kojin Bio, containing 1,750 JRU/mL of IL-2), Cosmedium 008 (Cosmo Bio, containing 1,750 JRU/mL of IL-2), FKCM101 (Fukoku, containing no IL-2 or 175 IU/mL of IL-2), CellGro SCGM medium (CellGenix, Iwai Chemical), X-VIVO15 medium (Lonza, Takara Bio), Gibco (registered trademark) CTS (registered trademark) AIM V (registered trademark) Medium (Thermo Fisher Scientific, serum-free medium of known composition for growth and manipulation of T cells and dendritic cells), CTS OpTmizer T Cell Expansion Basal Medium (Thermo Fisher Scientific, for growth and proliferation of human T lymphocytes), IMDM, MEM, DMEM, RPMI 1640, and so forth, but not limited to these. Preferred examples are the KBM501 medium, FKCM101, and Cosmedium 008. For the present invention, the expression that culture of cells (cells are cultured) means to maintain cells in a medium or a similar solution for a certain period of time for any purpose selected from the group consisting of maintaining cell viability, amplifying cells, and activating cells, unless especially stated. To carry out a treatment at a specific temperature for a certain period of time may be sometimes referred to as incubation (to incubate).

IL-2 may be added to the medium at such a concentration that the purpose of the invention can be achieved. The concentration of IL-2 may range from 2500 to 2813 IU/mL. IL-2 should preferably have a human amino acid sequence thereof, and be produced by a recombinant DNA technique for safety reasons. IL-2 concentration may be expressed in the national standard unit (Japan Reference Unit, JRU) and international unit (IU). 1 IU is approximately 0.622 JRU, and therefore 1,750 JRU/mL of the existing media is equivalent to approximately 2813 IU/mL.

Together with or instead of IL-2 described above, one selected from the group consisting of IL-12, IL-15, and IL-18 may be added at such a concentration that the purpose of the present invention can be achieved (Non-patent document 2, Leong JW et al., Biol. Blood Marrow Transplant, 20 (2014) 463-473). The concentration of each may be 1 pg/mL to 1 $\mu$g/mL irrespective of presence or absence or concentration of other cytokines. IL-2 preferably has a human amino acid sequence thereof, and be produced by recombinant DNA technique for safety reasons.

The medium may be supplemented with the subject's autologous serum, human type AB serum available from BioWhittaker and others, or donated blood human serum albumin available from the Japanese Red Cross Society. Autologous serum and human AB serum are preferably added at a concentration of 1 to 10%, and donated blood human serum albumin is preferably added at a concentration of 1 to 10%. Human platelet lysate (HPL) may be added together with or instead of serum. HPL is commercially available, and those of the UltraGRO™ series (AventaCell BioMedical), and so forth are commercially available. Sodium heparin may be further added to the medium, when HPL is used.

The medium may contain appropriate proteins, cytokines, antibodies, compounds, and other components, on condition that they do not impair the effectiveness of the NK cell culture. Cytokines may be IL-2, IL-12, IL-15, and IL-18 described above, as well as IL-3, IL-7, IL-21, stem cell factor (SCF), and/or FMS-like tyrosine kinase 3 ligand (Flt3L). All of these should preferably have human amino acid sequences thereof, and be produced by recombinant DNA technique for safety reasons.

The medium should preferably be a serum-free medium. The serum-free medium should preferably contain serum albumin, transferrin, and insulin. Serum-free media for culturing lymphocytes have been developed, and are commercially available, and they can be used for the present invention. One preferred example of serum-free medium is a basic medium supplemented with CTS Immune Cell SR (Thermo Fisher Scientific), which is commercially available as a composition that supports the proliferation of human T cells.

The medium may be replaced or replenished at any time after the start of culture, on condition that the desired culture effect is obtained, but the medium is preferably replaced or replenished every 3 to 5 days.

Culture vessels used for the culture include, but are not limited to, commercially available dishes, flasks, plates, and multi-well plates. Culture conditions are not particularly limited, so long as the culture effect of NK cells is not impaired, but culture conditions of 37°C, 5% $CO_2$, and saturated water vapor atmosphere are generally used. Culture period is not particularly limited, on condition that the desired culture effect is obtained.

Highly active NK cells, and so forth to which the present invention can be preferably applied include the following [1], [2], [3] and [4].

[1] NK cells having the following characteristics (1) and (2):

(1) CD16-positivity, high CD56 expression, and CD57-negativity, and
(2) NKG2C-positivity, NKG2A-negativity to low expression, and CD94-positivity.

The highly active NK cells of [1] may show high CD16 expression. The highly active NK cells of [1] may also have the following characteristics, regardless of whether or not they show high CD16 expression;
(3) Cytotoxic activity of 50% or higher when the NK cells are used as effector cells (E) for K562 cells as target cells (T) in co-culture at a mixing ratio (E:T) of 1:1.

The highly active NK cells of [1] can also be expressed as follows:
NK cells obtained by eliminating CD3-positive cells from peripheral blood mononuclear cells derived from a healthy human using CD3 beads (e.g., CliniMACS CD3, Miltenyi Biotech, catalog no. 130-017-601), LD column (e.g., Miltenyi Biotech, catalog no. 130-042-901), and a separation buffer (e.g., PBS containing 0.5% human AB serum (inactivated), and 2 mM EDTA), and culturing the obtained cell population for 14 days in an appropriate medium (e.g., Cosmedium 008 supplemented with 5% human AB serum (inactivated)), and having the following characteristics (1) and (3):

(1) CD16-positivity, CD56-high expression, and CD57-negativity, and
(3) Cytotoxic activity of 50% or higher when the NK cells are used as effector cells (E) for K562 cells as target cells (T) in co-culture at a mixing ratio (E:T) of 1:1.

For details of the characteristics, and more specific production methods of the highly active NK cells of [1], Japanese Patent Publication (Kokai) No. 2018-193303 can be referred to.

[2] The following cells:
CCR5-positive, CCR6-positive, CXCR3-positive and CD3-negative cells.

The cells of [2] may also show high CD11c expression.

The cells of [2] can also be expressed as follows

CCR5-positive, CCR6-positive, CXCR3-positive, integrin $\alpha$1-positive, integrin $\alpha$3-positive, integrin $\beta$3-negative, and CD3-negative cells. Alternatively, CCR5-positive, CCR6-positive, CXCR3-positive, highly CD11a-expressing, highly CD11c-expressing, and CD3-negative cells, of which high expressions are determined by comparison with the expressions in a population of NK cells obtained from peripheral blood that have not been substantially cultured.

According to the studies of the inventors of the present invention, the cells of [2] show extremely high cytotoxic activity against solid tumors forming tumor masses. For details of the characteristics, and more specific production methods of the highly active NK cells of [2], Japanese Patent Publication (Kokai) No. 2019-170176 can be referred to.

[3] Highly active NK cells obtainable by the following method:

To mononuclear cells obtained from fresh peripheral blood or frozen apheresis blood, add CD3 beads (e.g., CliniMACS CD3, Miltenyi Biotech, 130-017-601 (5 $\mu$L per 1 x $10^7$ cells)), and further CD34 beads (e.g., CliniMACS CD34, Miltenyi Biotech, 130-017-501 (2.5 $\mu$L per 1 x $10^7$ cells)) in the case of using mononuclear cells obtained from frozen apheresis blood, suspend them, incubate the suspension at 4°C for 15 minutes, then add a separation buffer (e.g., PBS containing 0.5% human type AB serum (inactivated at 56°C for 30 minutes), and 2 mM EDTA), suspend them well, and centrifuge the suspension. Remove the supernatant, and suspend the cells in 0.5 mL of the separation buffer in such a number that the cell number in one LD column (e.g., Miltenyi Biotech, 130-042-901) should be 1 x $10^8$ cells at the maximum. After adding 2 mL of the separation buffer to the LD column beforehand, add the cell suspension to the LD column, and collect eluate from the LD column. Add additional 1 mL of the separation buffer to the LD column, and collect eluate. After centrifuging the collected solution and removing the supernatant, suspend the cells in an appropriate medium (e.g., KBM501 medium containing 5% human AB serum (inactivated at 56°C for 30 minutes) or 5% UltraGRO (AventaCell, HPCPLCRL10) supplemented with 2 U/mL of sodium heparin) at a density of 5 x $10^5$ cells/mL in the case of using fresh peripheral blood, or 1 x $10^6$ cells/mL in the case of using frozen apheresis blood, and culture them up to day 14, with appropriately changing the medium.

For the specific production method of the highly active NK cells of [3], the section of Examples in this specification can be referred to.

[4] Cells obtained by obtaining any of the cells of [1] to [3] with adding any selected from the group consisting of IL-12, IL-15, and IL-18 at such a concentration that the purpose of the present invention can be achieved together with or instead of IL-2 at the time of culture. For the specific production method of such cells, Non-patent document 2 mentioned above can be referred to.

In the following explanations, the present invention may be explained with reference to the case of using highly active NK cells as an example, but those skilled in the art can also understand other cases using cells subjected to an in vitro activation operation using some cytokine in accordance with the explanations.

(Cytotoxic activity)

For the present invention, the term activity or cytotoxic activity used for highly active NK cells, and so forth refers to an ability of subject cells (effector cells, E) to lyse target cells (T), unless especially stated. Cytotoxic activity can be expressed as the percentage of target cells killed by effector cells, and is calculated in accordance with the following equation.

(Cell death observed in co-culture with effector cells - Spontaneous cell death (negative control))/(Maximum cell death (positive control) - Spontaneous cell death (negative control)) $\times$ 100

When cytotoxic activity is measured, in general, the mixing ratio of the effector cells to the target cells (E:T) and the time of co-culture of effector cells and target cells can be appropriately determined according to the degree of cytotoxic activity of the effector cells, etc., and depending on the types and the intensity of the activity of cells to be used. When NK cells are used as the effector cells, target cells may be, but are not limited to, K562 cells, acute myeloid leukemia cells, and chronic myeloid leukemia cells. The effector cells and target cells, and live cells and dead cells can be distinguished and quantified by using reagents such as antibodies labeled with a radioactive substance, fluorescent dye, or the like. When NK cells are used as the effector cells, the cytotoxic activity can be measured by using K562 cells as the target cells with the conditions of, for example, E:T of 1:0.05 to 10, preferably 1:0.1 to 5, and an incubation time of 0.5 to 18 hours, preferably 1 to 12 hours.

For the present invention, the expression that the activity of NK cells or the like is high means that the cytotoxic activity is 50% or higher when the target cells are K562 cells, and the cells are mixed at E:T of 2:1 and co-cultured for 1 to 3 hours, more specifically 2 hours, unless especially stated. The activity should be preferably 60% or higher, more preferably 70% or higher.

[Collection]

According to the present invention, prior to the freezing step described later, highly active NK cells or the like to be frozen are collected from the culture system. Collection can be performed by centrifuging the culture to separate the cells from the culture medium. If necessary, EDTA may be added at an appropriate concentration to the culture system to detach adhered cells from the surface of the culture vessel. The surface of the culture vessel may also be washed with an appropriate solution after the culture medium were collected to further obtain remaining cells. The obtained cells are washed with an appropriate solution and suspended in an appropriate solution, if necessary.

In the collection step, solutions such as culture medium, isotonic solution, and buffer may be used to detach and wash the cells. Examples of usable media include KBM501 medium, Cosmedium 008, FKCM101, CellGro SCGM medium, X-VIVO15 medium, Gibco (registered trademark) CTS (registered trademark) AIM V (registered trademark) Medium, CTS OpTmizer T Cell Expansion Basal Medium, IMDM, MEM, DMEM, and RPMI 1640. Isotonic solution refers to a solution with an osmolarity approximately equal to the osmolarity of body fluid (plasma) (285 $\pm$ 5 mOsm/L), and for the present invention, a solution with an osmolarity of 285 $\pm$ 13 mOsm/L. For example, the osmolality of Plasma-Lyte A is 294 mOsm/L, and that of PBS(-) is 280 $\pm$ 4 mOsm/L (freezing point depression method). Examples of usable isotonic solutions include Plasma-Lyte A (Baxter), saline (physiological saline), Ringer's solution (lactated Ringer's solution, acetated Ringer's solution, hydrogencarbonated Ringer's solution, etc.) and 5% glucose aqueous solution. Examples of buffers that can be used include phosphate-buffered saline (PBS), Tris-hydrochloric acid buffer, Trisacetic acid buffer, and HEPES buffer.

One of the preferred examples of the solution used in the collection step is a culture medium, more preferably a culture medium for human lymphocytes. The culture medium for human lymphocytes may contain human serum albumin, human transferrin, recombinant human insulin, and recombinant human IL-2. Preferred examples of such a medium are KBM501 medium, FKCM101, and Cosmedium 008. The KBM501 medium contains human serum albumin, human transferrin, recombinant human insulin, and recombinant human IL-2, but no other proteins. The KBM501 medium also contains antibiotics (kanamycin), $NaHCO_3$, L-glutamine, and pH adjuster.

In the collection step, use of PBS(-) may be undesirable, because it may reduce cell viability after thawing. PBS(-) typically contains 136.9 mM sodium chloride, 2.68 mM potassium chloride, 8.1 mM disodium hydrogenphosphate, and 1.47 mM potassium dihydrogenphosphate.

[Pretreatment]

For the present invention, prior to the freezing step described later, the highly active NK cells or the like to be frozen may be subjected to a pretreatment. The pretreatment means suspending the collected cells in a solution containing an additive. The pretreatment includes collecting the cells with a solution containing an additive.

The additive used for the pretreatment can be one selected from the group consisting of a bile acid and phenylbutyric acid. Examples of bile acid are tauroursodeoxycholic acid (TUDCA), ursodeoxycholic acid (UDCA), kenodeoxycholic acid, cholic acid, hyodeoxycholic acid, deoxycholic acid, 7-oxolithocholic acid, lithocholic acid, iododeoxycholic acid, iocholic acid, taurokenodeoxycholic acid, taurodeoxycholic acid, glycoursodeoxycholic acid, taurocholic acid, glycocholic acid, and analogues and derivatives thereof. Examples of phenylbutyric acid are 4-phenylbutyric acid (4-PBA), glyceryl tri-(4-PBA), phenylacetic acid, 2-POAA-OMe, 2-POAA-NO$_2$, 2-NOAA, pharmaceutically accep-table salts, analogues, derivatives and prodrugs thereof. Particularly preferred examples of the additive used for the pretreatment are any selected from the group consisting of TUDCA and 4-PBA.

When a bile acid is used as the additive for the pretreatment, the concentration thereof may be appropriately determined, but it is preferably 100 to 5000 $\mu$M, more preferably 200 to 2500 $\mu$M, further preferably 400 to 1000 $\mu$M. A concentration in such a range is particularly suitable when TUDCA is used. When a phenylbutyric acid is used as the additive for the pretreatment, the concentration thereof may be appropriately determined, but it is preferably 1 to 1000 $\mu$M, more preferably 5 to 500 $\mu$M, even more preferably 10 to 100 $\mu$M. A concentration in such a range is particularly suitable when 4-PBA is used.

Another example of the additive for the pretreatment is dimethyl sulfoxide (DMSO). The concentration thereof may be appropriately determined, but it is preferably 0.5 to 15%, more preferably 1 to 12.5%, further preferably 2 to 10%.

The solution for the pretreatment can be a solution such as medium, isotonic solution, and buffer, like the solution used for the collection. One preferred example of the solution used in the pretreatment is a culture medium, more preferably a medium for culture of human lymphocyte, further preferably KBM501 medium, FKCM101 or Cosmedium 008. The medium used for the pretreatment may also contain human serum albumin, human transferrin, recombinant human insulin, and recombinant human IL-2, and may contain antibiotics (kanamycin), NaHCO$_3$, L-glutamine, and pH adjuster.

The time for the pretreatment is not particularly limited. After the cells are suspended for the pretreatment, the suspension may be allowed to stand for several minutes to several hours, for example, 5 minutes to 4 hours, more preferably 30 minutes to 3 hours. The suspension may be allowed to stand at ambient temperature (e.g., 1 to 30°C, typically 15 to 25°C), and may be allowed to stand in a CO$_2$ incubator (e.g., 36 to 42°C, typically 37°C).

The cell density during the pretreatment may be appropriately determined, but should be a cell density suitable for cell maintenance. Specifically, the cell density is $1 \times 10^5$ to $1 \times 10^7$ cells/mL, preferably $2 \times 10^5$ to $5 \times 10^6$ cells/mL, more preferably $5 \times 10^5$ to $2 \times 10^6$ cells/mL.

In a particularly preferred embodiment, the pretreatment consists of suspending the cells in the KBM501 medium, FKCM101 or Cosmedium 008 supplemented with 400 to 1000 $\mu$M TUDCA or 10 to 100 $\mu$M 4-PBA at a cell density of $5 \times 10^5$ to $2 \times 10^6$ cells/mL. For this treatment, the cells are preferably incubated at 37°C and 5% CO$_2$ for 30 minutes to 3 hours.

Although the pretreatment is not essential for the present invention, the pretreatment of highly active NK cells or the like with the KBM501 medium supplemented with 4-PBA or TUDCA before freezing can improve the viability (also called recovery rate) after the cells are thawed compared with the case of not performing the pretreatment.

[Freezing]

According to the present invention, the collected and preferably pretreated cells are frozen by normal procedures. Specifically, the cell count and viability are checked as required, the supernatant is removed after centrifugation, and then the cells are suspended in a cryopreservation solution at an appropriate cell density. After dispensing the cell suspension into cryopreservation containers, it is frozen in a deep freezer at -80°C, and stored. If necessary, it is cryopreserved in a liquid nitrogen tank.

Cryopreservation solutions that can be used in the present invention can contain a sodium salt, potassium salt, sugar, hydrogencarbonate, carbonate, and cryoprotectant.

Sodium salts that can be used are not particularly limited so long as a sodium salt that produces sodium ion when it is dissolved in a solvent is used, and it can be an oxoacid salt, halide, oxide, hydroxide, inorganic salt, organic acid salt, or the like. One kind or a combination of two or more kinds of sodium salts may be used. For the present invention, sodium chloride is preferably used when one kind of salt is used, and sodium chloride and sodium citrate are preferably used when multiple kinds of salts are used. The sodium salt content is not particularly limited, but is preferably 0.01 to 5000 mM, more preferably 0.1 to 1000 mM, further preferably 1 to 300 mM, as the final concentration of total sodium ions contained in the cryopreservation solution.

Potassium salts that can be used are not particularly limited so long as a potassium salt that produces potassium ions when it is dissolved in a solvent is used, and it can be an oxoacid salt, halide, oxide, hydroxide, inorganic salt, organic acid salt, or the like. One kind of potassium salt may be used, or two or more kinds of potassium salts may be used in combination. Potassium chloride is preferably used in the present invention. The potassium salt content is not particularly limited, but as a final concentration of total potassium ions contained in the cryopreservation solution, it is preferably 0.01 to 5000 mM, more preferably 0.1 to 1000 mM, further preferably 1 to 100 mM.

Hydrogencarbonates that can be used are not particularly limited so long as a hydrogencarbonate that produces hydrogencarbonate ions when it is dissolved in a solvent is used, and salts with various cations can be used. Examples include, for example, ammonium hydrogencarbonate, potassium hydrogencarbonate, calcium hydrogencarbonate, sodium hydrogencarbonate, magnesium hydrogencarbonate, and so forth. Carbonates that can be used are not particularly limited so long as a carbonate that produces carbonate ions when it is dissolved in a solvent is used, and salts with a variety of cations can be used. Examples include ammonium carbonate, potassium carbonate, calcium carbonate, sodium carbonate, barium carbonate, magnesium carbonate, and so forth. One kind of these hydrogencarbonates and/or carbonates may be used, or two or more kinds of them may be used in combination. Sodium hydrogencarbonate is preferably used in the present invention. The content of hydrogencarbonate and/or carbonate is not particularly limited, but it is preferably 0.01 to 1000 mM, more preferably 0.1 to 500 mM, further preferably 1 to 100 mM, as the final concentration of total hydrogencarbonate and carbonate ions contained in the cryopreservation solution.

The concentration ratio of sodium ions to potassium ions (sodium ions/potassium ions) in the cryopreservation solution is preferably 1/1000 to 1000/1, more preferably 1/100 to 100/1, further preferably 1/10 to 100/1, further preferably 1/1 to 100/1, further preferably 10/1 to 50/1.

Usable sugars include monosaccharides, oligosaccharides, and sugar alcohols, such as glucose, galactose, fructose, mannose, xylose, arabinose as monosaccharides, trehalose, sucrose, maltose, lactose, cellobiose as oligosaccharides, xylitol, and sorbitol as sugar alcohols. One kind or a combination of two or more kinds of these sugars may be used. For the present invention, the sugar preferably consists of at least one kind of sugar selected from the group consisting of glucose, galactose, fructose, mannose, xylose, and arabinose, more preferably glucose. The content of the sugar in the cryopreservation solution is preferably 0.01 to 100 g/L, more preferably 0.1 to 100 g/L, further preferably 0.25 to 50 g/L.

Examples of usable cryoprotectants include dimethyl sulfoxide (DMSO), hydroxyethyl starch (HES), ethylene glycol, glycerol, and so forth. One kind or a combination of two or more kinds of cryoprotectants may be used. For the present invention, any selected from the group consisting of DMSO and hydroxyethyl starch is preferably used. When DMSO and hydroxyethyl starch are used together as the cryoprotectants, it is preferred that the total content thereof should be in the aforementioned range, and as for the respective concentrations, DMSO concentration is preferably 0.01 to 50%, more preferably 1 to 30%, further preferably 2 to 15%, and hydroxyethyl starch concentration is preferably 0.01 to 50%, more preferably 1 to 30%, further preferably 2 to 15%.

In a preferred embodiment of the present invention, in addition to the essential ingredients of the solution used in the method for cryopreservation of cells described above, the solution may further contain ingredients selected from the group consisting of proteins, magnesium salts and calcium salts. Proteins that can be used include, specifically, serum albumin, serum globulin, and so forth. Serum albumin includes human serum albumin, and bovine serum albumin. For the present invention, human serum albumin is preferred. The protein content in the cryopreservation solution is preferably 0.01 to 50%, more preferably 1 to 30%, further preferably 2 to 15%. Magnesium salts that can be used are not particularly limited so long as a magnesium salt that produces magnesium ions when it is dissolved in a solvent is used, and an oxoacid salt, halide, oxide, hydroxide, inorganic salt, organic acid salt, and so forth can be used. One kind of magnesium salt may be used, or two or more kinds of magnesium salts may be used in combination. Magnesium chloride is preferably used in the present invention. The magnesium salt content of the cryopreservation solution is not particularly limited, but it is preferably 0.01 to 10 mM, more preferably 0.1 to 5 mM, as the final concentration of total magnesium ions in the cryopreservation solution. Calcium salts that can be used are not particularly limited so long as a calcium salt that produces calcium ions when it is dissolved in a solvent is used, and an oxoacid salt, halide, oxide, hydroxide, inorganic salt, organic acid salt, or the like can be used. One kind of calcium salt may be used, or two more kinds of calcium salts may be used in combination. Calcium chloride is preferably used in the present invention. The calcium salt content of the cryopreservation solution is not particularly limited, but is preferably 0.01 to 10 mM, more preferably 0.1 to 5 mM, as the final concentration of total calcium ions in the cryopreservation solution. In addition to the above ingredients, the cryopreservation solution may also contain additional substances that are not injurious to cells, for example, vitamins, amino acids, and so forth. In addition to the above ingredients, the cryopreservation solution may also contain phosphate ions from the viewpoint of pH adjustment and buffering.

The osmolarity of the cryopreservation solution should be within such a range that the cells are not damaged during freezing. The osmolarity is, for example, 500 o 8000 mOsm/L, and may be 1000 to 7500 mOsm/L, 1500 to 7000 mOsm/L, or 1800 to 5000 mOsm/L, from the viewpoints of increasing the penetration of the ingredients into cells during freezing, and inhibition of ice crystal formation. The pH of the cryopreservation solution should be in such a range that the cells are not damaged, for example, 3.0 to 10.0, more preferably 4.5 to 9.0.

For the present invention, commercially available cryopreservation solutions may be used. Examples of products that can be used include the cryopreservation solutions for cells and tissues of the CellBanker series (STEM-CELLBANKER (registered trademark)), more specifically, STEM-CELLBANKER (ZENOAQ, CB045).

The cells are preferably in the logarithmic growth phase at the time of freezing.

The cell density at the time of freezing can be appropriately determined, but it is specifically $1 \times 10^6$ to $2 \times 10^8$ cells/mL, preferably $2 \times 10^6$ to $1 \times 10^8$ cells/mL, more preferably $1 \times 10^7$ to $5 \times 10^7$ cells/mL. In one of the preferred embodiments, the cells are stored at a cell density of $4 \times 10^7$ cells/mL in a 5-mL volume container. The characteristic of the highly active NK cells that they can be frozen at a high density at the time of freezing for shipping allows to make the product more compact, and contributes to lower shipping costs.

[Thawing and dilution]

In the present invention, cryopreserved cells can be thawed by various procedures. For example, such cells can be quickly thawed by incubating the cryopreservation container containing the cells in a warm bath at 37°C or the like, with shaking the container as required. Alternatively, cryopreserved cells can be spontaneously thawed by leaving them at room temperature without any active heating after they are taken out from the freezer. Making a frozen solid into a liquid may be referred to as unfreezing. At the time of or after thawing, the thawing cell cryoproduct can be diluted with an appropriate solution.

(Solution used for dilution)

The present invention relates to a solution for suspending cells for administration to humans, more specifically, a solution for diluting a cell cryoproduct (also referred to as solution for dilution or solution for thawing), which satisfies the following criteria:

(1) containing potassium ion,
(2) having a pH of 4.9 or higher,
(3) not containing chloride ions at a concentration of 135 mEq/L or higher,
(4) not containing glucose at a concentration of 5.55 mM or higher,
(5) not containing calcium ions at a concentration of 0.423 mM or higher,
(6) having an osmolarity of 200 to 396 mOsm, and
(7) containing any organic acid(s) selected from the group consisting of succinic acid, malic acid, lactic acid, citric acid, and acetic acid.

For the present invention, the expression that a solution does not contain a certain ingredient means that the ingredient is not detected by ordinary measurement methods, unless otherwise stated.

The solution for dilution contains any organic acid(s) selected from the group consisting of succinic acid, malic acid, lactic acid, citric acid, and acetic acid. These acids adjust the pH of the solution for dilution, but may be utilized by cells.

For the present invention, the expression that the solution contains any organic acid(s) selected from the group consisting of succinic acid, malic acid, lactic acid, citric acid, and acetic acid also means that the solution contains a pharmaceutically acceptable salt of any of these acids added during the preparation of the solution for dilution. In the solution for dilution, only one type or two or more types of these organic acids may be used.

In a preferred embodiment, the solution contains any organic acid(s) selected from the group consisting of succinic acid, lactic acid, citric acid, and acetic acid. In a particularly preferred embodiment, the solution for dilution contains succinic acid.

The amount of the organic acid selected from the group consisting of succinic acid, malic acid, lactic acid, citric acid, and acetic acid in the solution for dilution (when multiple types are contained, the total of the contents of them) is not particularly limited as long as the desired effect is obtained. The content of any of these organic acids in the solution for dilution can be, for example, 10 to 10,000 mg, and is preferably 50 to 5,000 mg, more preferably 200 to 3,000 mg, further preferably 400 to 1000 mg, in 200 mL.

In one embodiment, pH of the solution for dilution can be 4.7 or higher, and is preferably 4.9 or higher, more preferably 5.9 or higher, further preferably 6.3 or higher. The pH can also be 8.3 or lower, and is preferably 8.0 or lower, more preferably 7.9 or lower, further preferably 7.8 or lower.

The concentration of potassium ions in the solution for dilution is preferably 0.50 to 8.0 mM, more preferably 1.0 to 7.0 mM, further preferably 2.5 to 6.0 mM. Alternatively, it is preferred that the solution contains 0.496 to 5.96 mM potassium chloride.

In one preferred embodiment, the concentration of potassium ions in the solution for dilution is 4.00 mEq/L or higher. The maximum concentration is not particularly limited, but is, for example, 18.0 mEq/L or lower, preferably 15.0 mEq/L or lower, more preferably 10.0 mEq/L or lower, further preferably 7.50 mEq/L or lower.

In another preferred embodiment, the solution for dilution does not contain calcium ions, irrespective of the presence or absence of the other ingredients and concentrations thereof. In another preferred embodiment, the solution for dilution does not contain glucose, irrespective of the presence or absence of the other ingredients and concentrations thereof.

The ions contained in the solution for dilution may be derived from salts that can be used as pharmaceuticals. Such salts may be carbonates, hydrogencarbonates, oxoates, halides, oxides, hydroxides, inorganic salts, organic acid salts, or the like. One type of salt or a combination of two or more types of salts may be used.

The solution for dilution may contain a sodium salt, gluconate, and acetate. It may also contain a magnesium salt. The concentration of sodium ions in the solution for dilution is preferably 14.0 to 200 mM, more preferably 28.0 to 182 mM, further preferably 70.0 to 168 mM. Or the solution for dilution preferably contains 9.00 to 108 mM sodium chloride, 2.30 to 27.7 mM sodium gluconate, and 2.70 to 32.5 mM sodium acetate. The concentration of gluconate ions in the solution for dilution is preferably 2.3 to 32.3 mM, more preferably 4.6 to 29.9 mM, further preferably 12.5 to 27.7 mM. The concentration of acetate ions in the solution for dilution is preferably 2.7 to 37.8 mM, more preferably 5.4 to 35.1 mM, further preferably 13.5 to 32.5 mM.

The solution for dilution can be constituted by approved pharmaceuticals. Examples of such pharmaceuticals are listed below.

Generic name: Amizet

Bland name: Amizet B Infusion
Therapeutic classification: Comprehensive amino acid preparation
Composition (in 1 bag/200 mL)
Active ingredients: L-Isoleucine 1,700 mg, L-leucine 2,700 mg, lysine malate 2,432 mg (1,600 mg as L-lysine), L-methionine 780 mg, L-phenylalanine 1,540 mg, L-threonine 960 mg, L-tryptophan 320 mg, L-valine 1,800 mg, cysteine malate 310 mg (200 mg as L-cysteine), L-tyrosine 100 mg, L-arginine 2,220 mg, L-histidine 940 mg, L-alanine 1,720 mg, L-aspartic acid 100 mg, L-glutamic acid 100 mg, glycine 1,100 mg, L-proline 1,280 mg, and L-serine 840 mg
Additive: Succinic acid (pH adjuster) appropriate amount
Electrolyte: $Na^+$ and $Cl^-$ are not contained.

Generic name: MEYLON

Bland name: MEYLON Injection 8.4%
Common name: Sodium hydrogencarbonate
Composition (in 20mL)

Sodium hydrogencarbonate 1.68 g (8.4%)
Electrolyte concentration: $Na^+$ 1000 mEq/L, $HCO_3^-$ 1000 mEq/L

Generic name: K.C.L.

Bland name: K.C.L. Drip Injection 15%
Common name: Potassium chloride
Formulation: Potassium chloride formulation

Composition

Ingredients: Potassium chloride 3 g (15 w/v %, 2 M solution) [potassium (K) content 40 mEq (1573.36 mg)]
Additive: sodium riboflavin phosphate (riboflavin phosphate sodium) 6 mg

Generic name: Albumin (Kenketsu Albumin)

Brand name: Albumin (Kenketsu Albumin) 25% I.V. 12.5g/50mL
Therapeutic classification: Plasma fractionation product (human serum albumin preparation)
Composition (in 50mL)

Active ingredient: human serum albumin 12.5 g

Additives: acetyltryptophan 250.97 mg, sodium hydroxide 43.44 mg, sodium caprylate 169.75 mg

pH 6.4 to 7.4, osmotic ratio (ratio to physiological saline) 0.5 to 1.0

(Preferred embodiments)

One particularly preferred embodiment of the solution for dilution is the following.

| | |
|---|---|
| Albumin (Kenketsu Albumin) 25% | 20.0 mL |
| Physiological saline | 111.12 mL |
| Amizet | 19.08 mL |
| Distilled water | 56.68 mL |
| MEYLON | 1.6 mL |
| K.C.L. | 0.4 mL |
| pH=7.2 | |

Another preferred composition consists of the followings.

| | |
|---|---|
| Albumin (Kenketsu Albumin) 25% | 11.112 mL |
| Physiological saline | 111.12 mL |
| Amizet | 19.08 mL |
| Water | 56.68 mL |
| MEYLON | 1.6 mL |
| K.C.L. | 0.4 mL |
| pH=7.2 | |

Another preferred composition consists of the followings.

| | |
|---|---|
| Albumin (Kenketsu Albumin) 25% | 20.0 mL |
| Physiological saline | 111.12 mL |
| Amizet | 19.08 mL |
| Water | 56.68 mL |
| MEYLON | 1.6 mL |
| K.C.L. | 0.4 mL |
| pH=7.2 | |

Another preferred composition consists of the followings.

| | |
|---|---|
| Physiological saline | 11.7657 mL |
| * | 2.0202 mL |
| Water | 6.0014 mL |
| MEYLON | 0.1694 mL |
| K.C.L. | 0.0423 mL |
| Albumin (Kenketsu Albumin) 25% | 2.000 mL |
| pH=4.9 to 8.0 | |

* A solution containing L-cysteine or acetylcysteine and containing any organic acid(s) selected from the group consisting of succinic acid, lactic acid, citric acid, and acetic acid, which may contain other amino acids, solution obtained by adding any organic acid(s) selected from the group consisting of succinic acid, lactic acid, citric acid, and acetic acid to any of the formulations A to F described in the section of Examples, etc.

Cells suspended along with the solution for cryopreservation in the solution for dilution can be used as they are for administration. From the viewpoint of use for administration, it is preferred that the mixture of the cryopreservation solution and the solution for dilution should be isotonic (i.e., it has an osmolarity approximately equal to that of body fluid, specifically $285 \pm 13$ mOsm/L). Since the solution for cryopreservation is a hypertonic solution (e.g., 1500 to 7000 mOsm/L) in a preferred embodiment, the solution for dilution may be a low osmotic solution. Those skilled in the art can appropriately determine concentrations of the ingredients of the solution for dilution in consideration of the magnitude of dilution of the solution for cryopreservation with the solution for dilution.

In any composition of the solution for dilution, it is preferred that the solution for dilution does not contain serum at a concentration of 40% or higher, and it is more preferred that it does not contain serum at all. This is because if the solution for dilution contains serum, cell viability may be reduced.

Although the density of the cells suspended in the solution for dilution can be optionally chosen, it may be a cell density suitable for maintenance of the cells or cell density suitable for administration. Specifically, it is $1 \times 10^5$ to $1 \times 10^7$ cells/mL, preferably $2 \times 10^5$ to $5 \times 10^6$ cells/mL, more preferably $5 \times 10^5$ to $2 \times 10^6$ cells/mL.

The cells can be maintained in the solution for dilution for a relatively long period of time. After suspending the cells in the solution for dilution, the suspension may be allowed to stand for several minutes to several hours, e.g., 5 minutes to 6 hours, more preferably 30 minutes to 4 hours. They may be allowed to stand at ambient temperature (e.g., 1 to 30°C, typically 15 to 25°C), or in a $CO_2$ incubator (e.g., 36 to 42°C, typically 37°C).

By using such a solution for dilution, viability of frozen highly active NK cells etc. can be maintained at a high level. In addition, the cytotoxic activity of the frozen highly active NK cells etc. can be maintained at a high level. For viability, the percentage of viable cell count may be expressed as A, 70% or higher; B, 50% or higher and lower than 70%; and C, lower than 50%. For cytotoxic activity, the percentage of target cells killed by effector cells may be expressed as A, 70% or higher; B, 50% or higher and lower than 70%; and C, lower than 50%. For both items, if the condition A or B is achieved, it can be said that the object has been achieved.

[Use in pharmaceutical compositions]

The present invention provides a pharmaceutical composition containing highly active NK cells, which have been collected by an appropriate method, pretreated as required, and cryopreserved, and so forth.

The pharmaceutical composition provided by the present invention can be used for the treatment and/or prevention of various diseases susceptible to highly active NK cells, and so forth. Examples of such diseases are cancers and infectious diseases, and specifically, they include, but not limited to, skin cancer, oral cancer, gallbladder cancer, bile duct cancer, lung cancer, liver cancer, stomach cancer, colon cancer, pancreatic cancer, kidney cancer, ovarian cancer, bladder cancer, prostate cancer, neuroblastoma, leukemia, and infectious diseases caused by viruses, bacteria or the like. The inventors of the present invention confirmed the effect of use of cells frozen and thawed by the method of the present invention on animal models of colon cancer, which should otherwise die within 30 days without any treatment.

A cell therapy using the pharmaceutical composition of the present invention may be performed solely, or in combination with surgical therapy, chemotherapy, radiation therapy, antibody drugs, and so forth.

The characteristics of one embodiment of the pharmaceutical composition provided by the present invention are shown below.

(Dosage form)

Injection (Injectable cell suspension for parenteral administration ) (also referred to herein infusion, intravenous infusion, infusion composition, or pharmaceutical composition for infusion)

(Ingredient and content)

Component cells: Highly active NK cells, etc.
Content: $6 \times 10^6$ to $4.8 \times 10^9$ cells/60 kg

(Sub-ingredients)

Complex electrolyte solution 10 to 45%
Sodium chloride solution 10 to 45%
20 to 30% Human serum albumin solution 5 to 30%

Dimethyl sulfoxide 2 to 15%
Others
or
Cryopreservation solution acceptable as a pharmaceutical additive 100%

(Preparation method)

The frozen composition is thawed in a thermostatic bath at 37°C or the like until it is completely thawed. Immediately after the thawing, the cells are aseptically suspended in a separately prepared isotonic solution acceptable as a pharmaceutical additive.

(Stability after thawing)

The shelf life of the composition after thawing is 6 hours, preferably 4 hours, when it is stored at room temperature.

Examples

[Methods commonly used in Examples]

A) Method for culturing GAIA-102

Frozen apheresis blood (Cellero) as the raw material was thawed, and then the cells were washed and concentrated by using Lovo Cell Processing System (Fresenius Kabi) to obtain PBMCs.

CD3-Positive and CD34-positive cells were removed from the resulting PBMCs by using CliniMACS Prodigy (registered trademark, Miltenyi Biotec), and the cells were eluted with KBM501 medium[*1]. The numbers of the cells in the eluate was counted, and the total cell count was calculated. The culture was performed by using an adhesion culture bag 640 cm$^2$, to which 200 mL per bag of a cell suspension prepared in the KBM501 medium containing Simulect (Novartis Pharma) and Prograf (Astellas Pharma) at a density of $5 \times 10^5$ cells/mL was inoculated, in a $CO_2$ incubator (37°C, 5% $CO_2$). On day 9 of the culture, the KBM501 medium containing Simulect was further added to make the final volume 650 mL per bag, and the incubation was continued until day 14.

*1: KBM501 (Kohjin Bio) supplemented with 5% UltraGRO (AventaCell, HPCPLCRL10) and 2 U/mL heparin sodium (Nipro).

B) Method for collecting GAIA-102

On day 14 of the culture, the culture medium was collected, and 1 mM EDTA was added to the culture bag to detach the adhered cells. The total volume of the collected culture medium and the detached cells were centrifuged, and the cells were washed with and resuspended in the KBM501 medium.

C) Method for freezing GAIA-102

The number of viable cells of GAIA-102 obtained by the procedures described as the methods for culturing and collecting GAIA-102 was counted, and $2 \times 10^8$ cells were suspended in 5 mL of HSC-BANKER (ZENOAQ, CB071) and frozen at -80°C.

D) Method for measuring viability based on 7-AAD staining

The GAIA-102 cells thawed under each condition were prepared on a 96-well plate (IWAKI, 4870-800SP) at a density of $1 \times 10^5$ cells/well, and centrifuged, the supernatant was removed, then a 7-AAD solution (Beckman Coulter, A07704) diluted with PBS (Nacalai Tesque, 14294-95) was added to suspend the cells, and the plate was incubated at room temperature for 20 minutes. After the staining, measurement of the cells was performed by using a flow cytometer (BD LSR Fortessa, BD Biosciences), the results were analyzed with FlowJo software, and the viability was calculated from the 7-AAD-positive rate.

E) Method for measuring cytotoxic activity against tumor cells

For the measurement of cytotoxic activity, a group of the GAIA-102 cells thawed and reacted with the K562 cells, a group solely consisting of the K562 cells as a negative control, and a group of the K562 cells fixed with 10% formalin as a positive control were prepared.

<<GAIA-102>>

The GAIA-102 cells were thawed and diluted under each condition, and a necessary amount of the cells were taken on the basis of the number of viable cells at the time of freezing, and then prepared at a density of $1 \times 10^6$ cells/ mL in 10% FBS/RPMI 1640.

<<K562>>

The K562 cells were suspended in serum-free RPMI 1640 medium, stained by using PKH26 Red Fluorescent Cell Linker Kit, and then prepared at a density of $2 \times 10^6$ cells/mL in 10% FBS/RPMI 1640.

The GAIA-102 cells and K562 cells were added to and mixed in wells of 96-well plate (IWAKI, 4870-800SP) at a cell ratio of 1:1, 2:1, or 8:1, and allowed to react at 37°C for 2 hours under 5% $CO_2$. After the reaction, the plate was centrifuged (500 x g, 5 minutes), the supernatant was removed, then a 7-AAD solution diluted with PBS was added to suspend the cells, and the suspension was incubated at room temperature for 20 minutes. Measurement was performed by using a flow cytometer, and the results were analyzed with the FlowJo software to calculate the cytotoxic activity rate (% Lysis)[*2].

2*: Cytotoxic activity rate = (K562 Cell dead cell rate - Negative control dead cell rate)/(Positive control dead cell rate - Negative control dead cell rate) $\times$ 100

F) Method for calculating corrected values of cytotoxic activity rate by statistical analysis

Calculation values for ET ratio = 1 and 2 were calculated by nonlinear regression analysis using JMP (registered trademark) Pro statistical analysis software from the cytotoxic activity rates calculated from the results for 4 or more of ET ratios (including 0).

Reagents and infusion agents used

The following reagents and infusion agents were used in the verification of solutions for thawing.

Plasma-Lyte A (Baxter)

Physiological saline, Otsuka Normal Saline Injection (Otsuka Pharmaceutical Co., Ltd.)
Water (Nacalai Tesque)
MEYLON Injection 8.4% (Otsuka Pharmaceutical Co., Ltd.)
K.C.L. Drip Injection 15% (Maruishi Pharmaceutical Co., Ltd.)
Albumin (Kenketsu Albumin) 25% I.V. Injection (Japan Pharmaceutical Co., Ltd.)
L-(-)-Malic acid (Nacalai Tesque, 21030-44)
Succinic acid (Nacalai Tesque, 32402-92)
Citric acid monohydrate (Nacalai Tesque, 09106-02)
Lactic acid (Nacalai Tesque, 20006-62)
Acetic acid (Nacalai Tesque, 00212-85)
L(+)-Ascorbic acid (Nacalai Tesque, 03420-52)

[Example 1]

Solutions having the composition shown in the following table were prepared and used as solutions for thawing (1). Frozen GAIA-102 cells were thawed on a water bath at 37°C, and then diluted 45-fold with each of the solutions for thawing (1) or 41-fold with Plasma-Lyte A as a control. After thawing, the samples were allowed to stand at room temperature for 3 hours, and then the cytotoxic activity was measured.

[Table 1-1]

| Ingredient | Amount (mg) in 200 ml | | | |
|---|---|---|---|---|
| | Modified formulation A | Modified formulation B | Modified formulation C | Modified formulation D |
| L-Isoleucine | 1,700 | 1,700 | 1,700 | 1,700 |
| L-Leucine | 2,700 | 2,700 | 2,700 | 2,700 |
| Lysine acetate | 2,257 | 2,257 | - | 2,257 |
| L-Methionine | 780 | 780 | 780 | 780 |
| L-Phenylalanine | 1,540 | 1,540 | 1,540 | 1,540 |
| L-Threonine | 960 | 960 | 960 | 960 |
| L-Tryptophan | 320 | 320 | 320 | 320 |
| L-Valine | 1,800 | 1,800 | 1,800 | 1,800 |
| L-Cysteine | 200 | - | - | - |
| Acetylcysteine | - | 269 | - | - |
| L-Tyrosine | 100 | 100 | 100 | 100 |
| L-Arginine | 2,220 | 2,220 | 2,220 | 2,220 |
| L-Histidine | 940 | 940 | 940 | 940 |
| L-Alanine | 1,720 | 1,720 | 1,720 | 1,720 |
| L-Aspartic acid | 100 | 100 | 100 | 100 |
| L-Glutamic acid | 100 | 100 | 100 | 100 |
| Glycine | 1,100 | 1,100 | 1,100 | 1,100 |
| L-Proline | 1,280 | 1,280 | 1,280 | 1,280 |
| L-Serine | 840 | 840 | 840 | 840 |
| Water for Injection | Appropriate volume | Appropriate volume | Appropriate volume | Appropriate volume |

[Table 1-2]

| <Compositions of solutions for thawing (1)> | |
|---|---|
| | Volume (mL) |
| Physiological saline | 11.7657 |
| Formulation A/B/C/D*3 | 2.0202 |
| Water | 6.0014 |
| MEYLON | 0.1694 |
| K.C.L. | 0.0423 |
| Albumin (Kenketsu Albumin) 25% | 2.000 |

*3 Four groups of Formulation A as it is, Formulation B as it is, Formulation C as it is, and Formulation D as it is, The results are shown in Fig. 1. High cytotoxic activity against the tumor cells comparable to that observed with Plasma-Lyte A was observed when Formulations A and B were used,

[Example 2]

Malic acid, succinic acid, or malic acid and succinic acid were added to Formulations A and B to obtain solutions of pH 6.9 to 7.4. Then, solutions having the composition shown in the following table were prepared as solutions for thawing (2), and pH values thereof were measured after the preparation. Frozen GAIA-102 cells were thawed on a water bath at 37°C, and then diluted 45-fold with each of the solutions for thawing (2) or 41-fold with Plasma-Lyte A as a control. After thawing, the samples were allowed to stand at room temperature for 3 hours, and then the cytotoxic activity was measured.

[Table 2-1]

|  | Formulation A or B | Malic acid | Succinic acid |
|---|---|---|---|
| Formulation A | 40mL | - | - |
| Formulation A + malic acid | 40mL | 0.14g | - |
| Formulation A + succinic acid | 40mL | - | 0.14g |
| Formulation A + malic acid + succinic acid | 40mL | 0.02g | 0.12g |
| Formulation B | 40mL | - | - |
| Formulation B + malic acid | 40mL | 0.12g | - |
| Formulation B + succinic acid | 40mL | - | 0.12g |
| Formulation B + malic acid + succinic acid | 40mL | 0.02g | 0.08g |

[Table 2-2]

| <Compositions of solutions for thawing (2)> | |
|---|---|
|  | Volume (mL) |
| Physiological saline | 11.7657 |
| Formulation A/B*4 | 2.0202 |
| Water | 6.0014 |
| MEYLON | 0.1694 |
| K.C.L. | 0.0423 |
| Albumin (Kenketsu Albumin) 25% | 2.000 |

*4 Eight groups of Formulation A as it is (pH 7.9), Formulation A + malic acid (pH 7.3), Formulation A + succinic acid (pH 7.2), Formulation A + malic acid + succinic acid (pH 7.3), Formulation B as it is (pH 7.9), Formulation B + malic acid (pH 7.4), Formulation B + succinic acid (pH 7.2), and Formulation B + malic acid + succinic acid (pH 7.4), pH values of the solutions for thawing after preparation are shown in the parentheses.

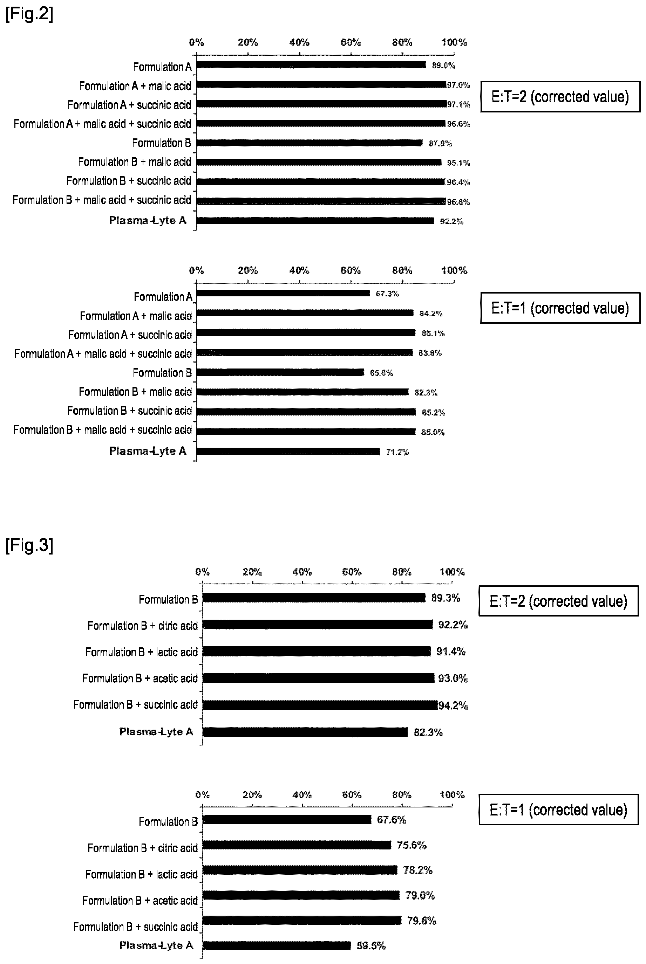

The results are shown in Fig. 2. Higher cytotoxic activity against the tumor cells was observed when malic acid and succinic acid were used.

[Example 3]

Citric acid monohydrate, lactic acid, acetic acid, and succinic acid were added to Formulation B to obtain solutions of pH 6.9 to 7.7. Then, solutions having the composition shown in the following table were prepared as solutions for thawing (3), and pH values thereof were measured after the preparation. Frozen GAIA-102 cells were thawed on a water bath at 37°C, and then diluted 45-fold with each of the solutions for thawing (3) or 41-fold with Plasma-Lyte A as a control. After thawing, the samples were allowed to stand at room temperature for 3 hours, and then the cytotoxic activity was measured.

[Table 3-1]

|  | Formulation B |  |
|---|---|---|
| Formulation B | 40mL | - |
| Formulation B + citric acid monohydrate | 40mL | 0.128g |

(continued)

|  | Formulation B |  |
|---|---|---|
| Formulation B + lactic acid | 40mL | 160μL |
| Formulation B + acetic acid | 40mL | 80μL |
| Formulation B + succinic acid | 40mL | 0.12g |

[Table 3-2]

| <Compositions of solutions for thawing (3)> |  |
|---|---|
|  | Volume (mL) |
| Physiological saline | 11.7657 |
| Formulation B*5 | 2.0202 |
| Water | 6.0014 |
| MEYLON | 0.1694 |
| K.C.L. | 0.0423 |
| Albumin (Kenketsu Albumin) 25% | 2.000 |

*5 Five groups of Formulation B as it is (pH 8.0), Formulation B + citric acid monohydrate (pH 7.4), Formulation B + lactic acid (pH 7.4), Formulation B + acetic acid (pH 7.5), and Formulation B + succinic acid (pH 7.4), pH values of the solutions for thawing after preparation are shown in the parentheses.

The results are shown in Fig. 3. Higher cytotoxic activity against the tumor cells was observed when citric acid, lactic acid, and acetic acid were used, like when succinic acid was used.

[Example 4]

Ascorbic acid and succinic acid were added to Formulation B to obtain solutions of pH 7.5 or 7.8. Then, solutions having the composition shown in the following table were prepared as solutions for thawing (4), and pH values thereof were measured after the preparation. Frozen GAIA-102 cells were thawed on a water bath at 37°C, and then diluted 45-fold with each of the solutions for thawing (4) or 41-fold with Plasma-Lyte A as a control. After thawing, the samples were allowed to stand at room temperature for 3 hours, then the cytotoxic activity was measured, and viability was measured by 7-AAD staining.

[Table 4-1]

| Ingredient | Amount (mg) in 200 ml | |
|---|---|---|
|  | Formulation E | Formulation F |
| L-Isoleucine | 1,700 | 1,700 |
| L-Leucine | 2,700 | 2,700 |
| Lysine acetate | 2,257 | 2,257 |
| L-Methionine | 780 | 780 |
| L-Phenylalanine | 1,540 | 1,540 |
| L-Threonine | 960 | 960 |
| L-Tryptophan | 320 | 320 |
| L-Valine | 1,800 | 1,800 |
| Acetylcysteine | 269 | 269 |
| L-Tyrosine | 100 | 100 |

(continued)

| Ingredient | Amount (mg) in 200 ml | |
|---|---|---|
| | Formulation E | Formulation F |
| L-Arginine | 2,220 | 2,220 |
| L-Histidine | 940 | 940 |
| L-Alanine | 1,720 | 1,720 |
| L-Aspartic acid | 100 | 100 |
| L-Glutamic acid | 100 | 100 |
| Glycine | 1,100 | 1,100 |
| L-Proline | 1,280 | 1,280 |
| L-Serine | 840 | 840 |
| Succinic acid (pH adjuster) | Appropriate amount | Appropriate amount* |
| Water for Injection | Appropriate volume | Appropriate volume |
| pH | 7.0 | 7.5 |
| *700 mg/200 mL or so. | | |

[Table 4-2]

| <Compositions of solutions for thawing (4)> | |
|---|---|
| | Volume (mL) |
| Physiological saline | 11.7657 |
| Formulation B/E/F[6] | 2.0202 |
| Water | 6.0014 |
| MEYLON | 0.1694 |
| K.C.L. | 0.0423 |
| Albumin (Kenketsu Albumin) 25% | 2.000 |

*6 Five groups of Formulation B as it is (pH 8.2), Formulation B + ascorbic acid (pH 7.6), Formulation B + succinic acid (pH 7.9), Formulation E as it is (pH 7.7), and Formulation F as it is (pH 7.8), pH values of the solutions for thawing after preparation are shown in the parentheses.

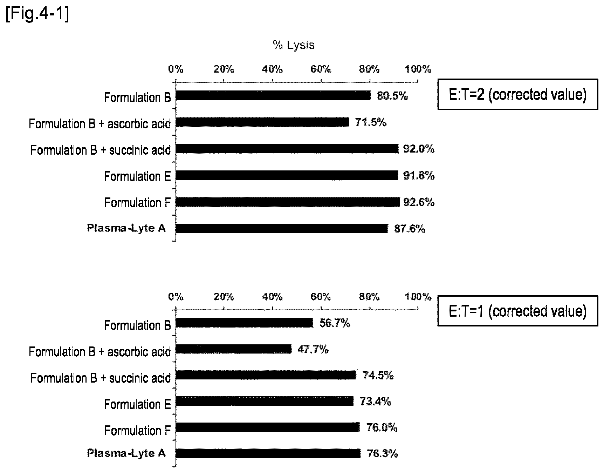

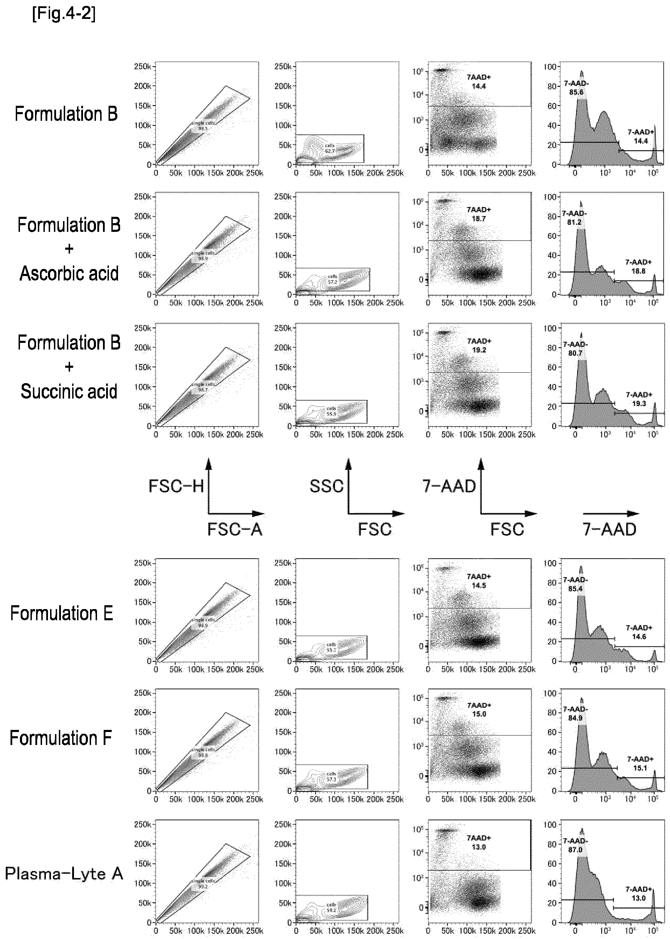

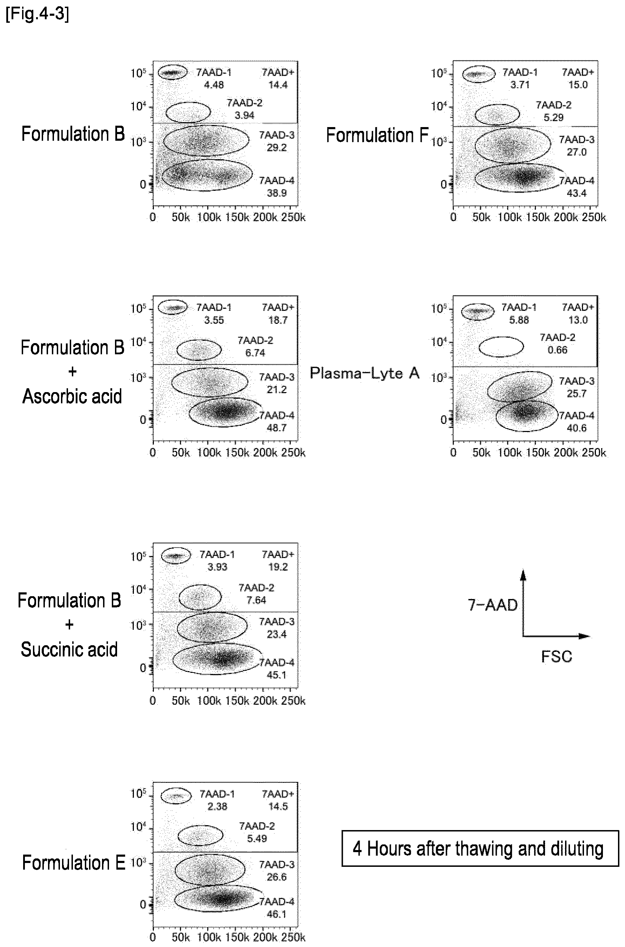

The results are shown in Fig. 4-1. When ascorbic acid was used, cytotoxic activity against the tumor cells as high as that observed when succinic acid was used could not be observed. The results of viability evaluation based on 7-AAD staining are shown in Figs. 4-2 and 4-3.

[Example 5]

Succinic acid was added to Formulation F to obtain solutions of pH 5.9 to 7.1. Then, solutions having the composition shown in the following table were prepared as solutions for thawing (5), and pH values thereof were measured after the preparation. Frozen GAIA-102 cells were thawed on a water bath at 37°C, and then diluted 45-fold with each of the solutions for thawing (5) or 41-fold with Plasma-Lyte A as a control. After thawing, the samples were allowed to stand at room temperature for 3 hours, then the cytotoxic activity was measured, and viability was measured by 7-AAD staining.

[Table 5-1]

| Formulation F | Succinic acid | pH |
|---|---|---|
| 20mL | - | 8.0 |

(continued)

| Formulation F | Succinic acid | pH |
|---|---|---|
| 20mL | 0.02g | 7.1 |
| 20mL | 0.04g | 6.3 |
| 20mL | 0.06g | 5.9 |

[Table 5-2]

| <Compositions of solutions for thawing (5)> | |
|---|---|
| | Volume (mL) |
| Physiological saline | 11.7657 |
| Formulation F*7 | 2.0202 |
| Water | 6.0014 |
| MEYLON | 0.1694 |
| K.C.L. | 0.0423 |
| Albumin (Kenketsu Albumin) 25% | 2.000 |

*7 Four groups of Formulation F as it is (pH 7.8), Formulation F + succinic acid (pH 7.5), Formulation F + succinic acid (pH 7.4), and Formulation F + succinic acid (pH 7.3), pH values of the solutions for thawing after preparation are shown in the parentheses.

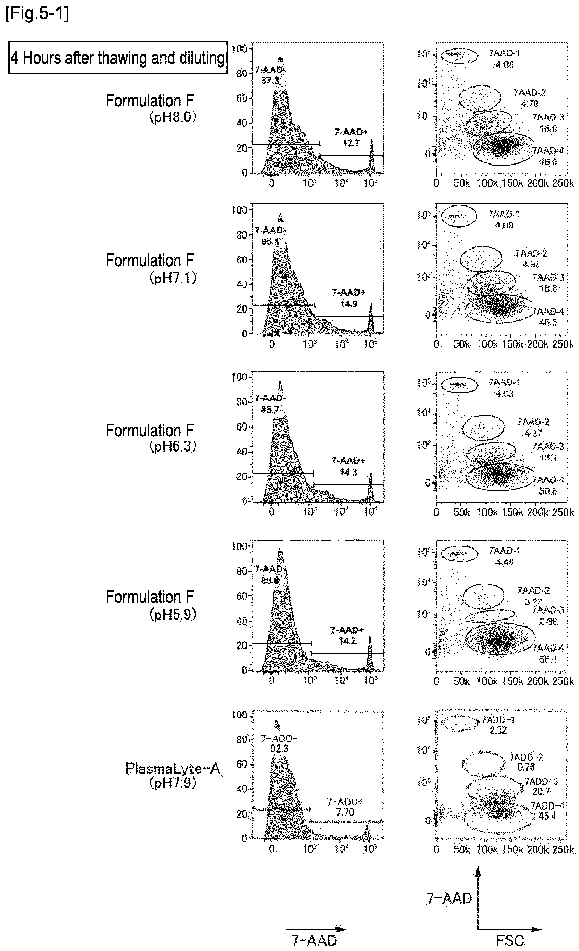

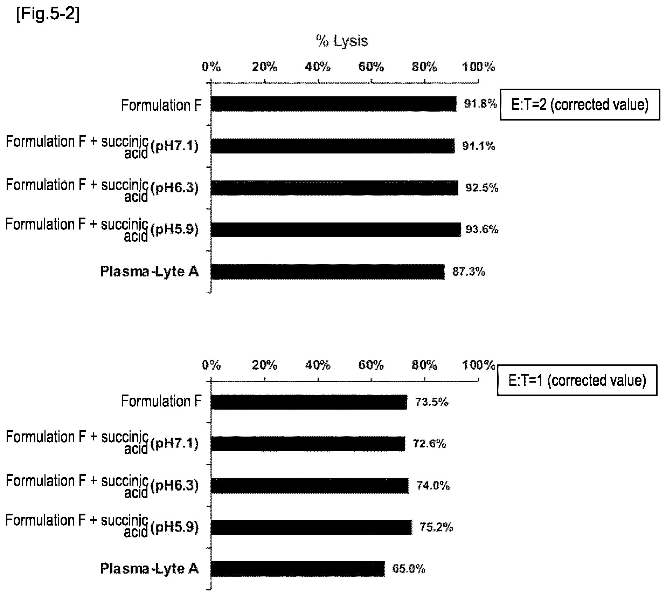

The results are shown in Figs. 5-1 and 5-2. When succinic acid was used, favorable results were obtained over a wide pH range. Viability was 87.3% for Formulation F as it is (pH 7.8), 85.1% for Formulation F + succinic acid (pH 7.5), 85.7% for formulation F + succinic acid (pH 7.4), and 85.8% for Formulation F + succinic acid (pH 7.3). The viability for PlasmaLyte-A (pH 7.9) was 92.3%.

[Example 6]

Succinic acid was added to Formulation F to obtain solutions of pH 4.9 to 5.9. Then, solutions having the compositions shown in the following below were prepared as solutions for thawing (6), and pH values thereof were measured after the preparation. Frozen GAIA-102 cells were thawed on a water bath at 37°C, and then diluted 45-fold with each of the solutions for thawing (6) or 41-fold with Plasma-Lyte A as a control. After thawing, the samples were allowed to stand at room temperature for 3 hours, then the cytotoxic activity was measured, and viability was measured by 7-AAD staining.

[Table 6-1]

| Formulation F | Succinic acid | pH |
|---|---|---|
| 20mL | - | 7.9 |
| 20mL | 0.06g | 5.9 |
| 20mL | 0.09g | 5.4 |
| 20mL | 0.17g | 4.9 |

[Table 6-2]

| <Compositions of solutions for thawing (6)> | |
|---|---|
| | Volume (mL) |
| Physiological saline | 11.7657 |

(continued)

| <Compositions of solutions for thawing (6)> | |
| --- | --- |
| | Volume (mL) |
| Formulation F*8 | 2.0202 |
| Water | 6.0014 |
| MEYLON | 0.1694 |
| K.C.L. | 0.0423 |
| Albumin (Kenketsu Albumin) 25% | 2.000 |

*8 Four groups of Formulation F as it is (pH 7.7), Formulation F + succinic acid (pH 7.2), Formulation F + succinic acid (pH 6.9), and Formulation F + succinic acid (pH 6.1), pH values of the solutions for thawing after preparation are shown in the parentheses.

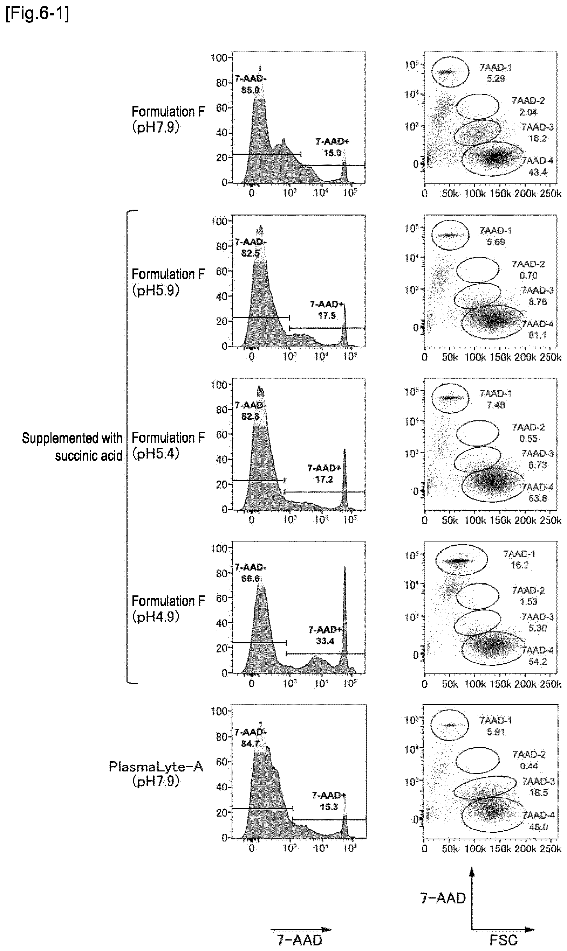

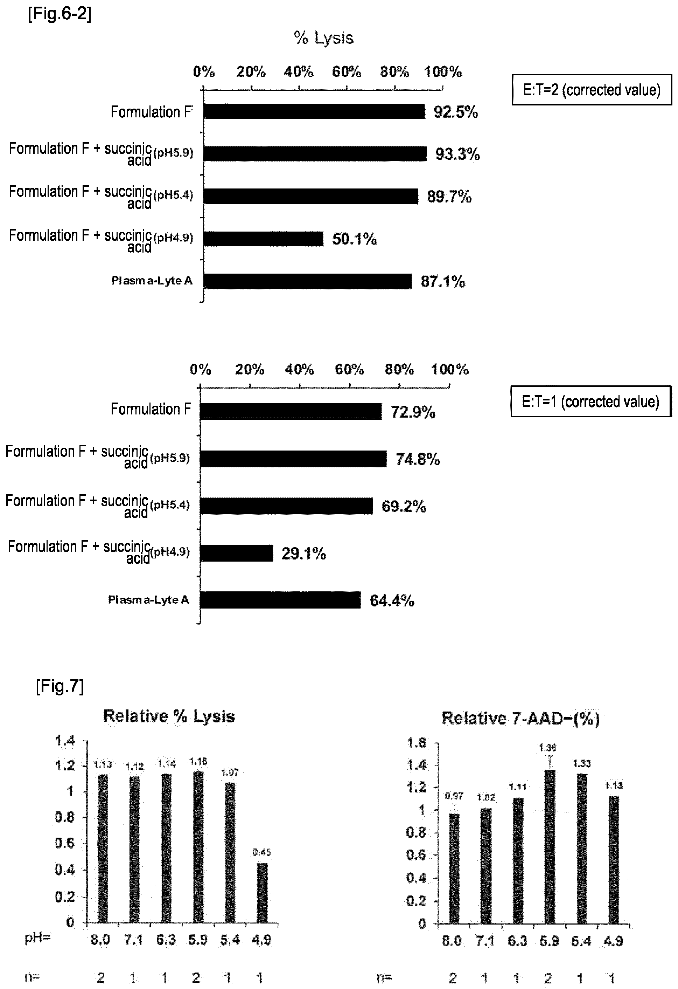

The results are shown in Figs. 6-1 and 6-2. The viability was 85.0% for Formulation F as it is (pH 7.7), 82.5% for Formulation F + succinic acid (pH 7.2), 82.8% for Formulation F + succinic acid (pH 6.9), and 66.6% for Formulation F + succinic acid (pH 6.1). The viability for Plasma-Lyte A (pH 7.9) was 84.7%.

[Summary of Examples 5 and 6]

Relative % lysis (E:T=1, corrected value) and 7-AAD-4 gate (%) based on those obtained with PlasmaLyte-A taken as 1.0 are summarized in Fig. 7.

## Claims

1. A solution for suspending cells for administration to humans, which satisfies the following:

   (1) contains potassium ions;
   (2) has a pH of 4.9 or higher;
   (3) does not contain chloride ions at a concentration of 135 mEq/L or higher;
   (4) does not contain glucose at a concentration of 5.55 mM or higher;
   (5) does not contain calcium ions at a concentration of 0.423 mM or higher;
   (6) has an osmotic pressure of 200 to 396 mOsm; and
   (7) contains any organic acid(s) selected from the group consisting of succinic acid, malic acid, lactic acid, citric acid, and acetic acid.

2. The solution according to claim 1, wherein the organic acid (7) is succinic acid.

3. The solution according to claim 1, which further satisfies (8) containing L-cysteine or acetylcysteine.

4. The solution according to claim 1, which further satisfies (9) containing albumin.

5. The solution according to any one of claims 1 to 4, which is for diluting a frozen product containing cells for administration to humans or a thawed product thereof.

6. The solution according to any one of claims 1 to 4, which satisfies

   (1) contains potassium ions at a concentration of 4.00 mEq/L or higher;
   (4) does not contain glucose; and
   (5) does not contain calcium ions.

7. The solution according to any one of claims 1 to 4, which is a pharmaceutical composition.

8.  A pharmaceutical composition containing the solution according to any one of claims 1 to 4 and a population of cells for administration to humans, wherein the cells for administration to humans are lymphocytes.

9.  A pharmaceutical composition containing the solution according to any one of claims 1 to 4 and a population of cells for administration to humans, wherein the cells for administration to humans are highly active, NK cell-like allogeneic CD3-negative cells.

10. The pharmaceutical composition according to claim 9, wherein the population of highly active, NK cell-like allogeneic CD3-negative cells has undergone a freezing process.

11. A method for providing a pharmaceutical composition for infusion containing cells for administration to a human, which comprises the step of thawing cryopreserved cells and suspending the thawed cells in the solution according to any one of claims 1 to 4 to prepare a pharmaceutical composition for infusion.

[Fig.1-1]

[Fig. 1-2]

[Fig.2]

E:T=2 (corrected value)

| Formulation | % |
|---|---|
| Formulation A | 89.0% |
| Formulation A + malic acid | 97.0% |
| Formulation A + succinic acid | 97.1% |
| Formulation A + malic acid + succinic acid | 96.6% |
| Formulation B | 87.8% |
| Formulation B + malic acid | 95.1% |
| Formulation B + succinic acid | 96.4% |
| Formulation B + malic acid + succinic acid | 96.8% |
| Plasma-Lyte A | 92.2% |

E:T=1 (corrected value)

| Formulation | % |
|---|---|
| Formulation A | 67.3% |
| Formulation A + malic acid | 84.2% |
| Formulation A + succinic acid | 85.1% |
| Formulation A + malic acid + succinic acid | 83.8% |
| Formulation B | 65.0% |
| Formulation B + malic acid | 82.3% |
| Formulation B + succinic acid | 85.2% |
| Formulation B + malic acid + succinic acid | 85.0% |
| Plasma-Lyte A | 71.2% |

[Fig.3]

E:T=2 (corrected value)

| Formulation | % |
|---|---|
| Formulation B | 89.3% |
| Formulation B + citric acid | 92.2% |
| Formulation B + lactic acid | 91.4% |
| Formulation B + acetic acid | 93.0% |
| Formulation B + succinic acid | 94.2% |
| Plasma-Lyte A | 82.3% |

E:T=1 (corrected value)

| Formulation | % |
|---|---|
| Formulation B | 67.6% |
| Formulation B + citric acid | 75.6% |
| Formulation B + lactic acid | 78.2% |
| Formulation B + acetic acid | 79.0% |
| Formulation B + succinic acid | 79.6% |
| Plasma-Lyte A | 59.5% |

[Fig.4-1]

% Lysis

E:T=2 (corrected value)

| Formulation | % Lysis |
|---|---|
| Formulation B | 80.5% |
| Formulation B + ascorbic acid | 71.5% |
| Formulation B + succinic acid | 92.0% |
| Formulation E | 91.8% |
| Formulation F | 92.6% |
| Plasma-Lyte A | 87.6% |

E:T=1 (corrected value)

| Formulation | % Lysis |
|---|---|
| Formulation B | 56.7% |
| Formulation B + ascorbic acid | 47.7% |
| Formulation B + succinic acid | 74.5% |
| Formulation E | 73.4% |
| Formulation F | 76.0% |
| Plasma-Lyte A | 76.3% |

[Fig.4-2]

[Fig.4-3]

Formulation B

Formulation F

Formulation B
+
Ascorbic acid

Plasma-Lyte A

Formulation B
+
Succinic acid

7-AAD

FSC

Formulation E

4 Hours after thawing and diluting

[Fig.5-1]

4 Hours after thawing and diluting

Formulation F (pH8.0)

Formulation F (pH7.1)

Formulation F (pH6.3)

Formulation F (pH5.9)

PlasmaLyte-A (pH7.9)

[Fig.5-2]

[Fig.6-1]

[Fig.6-2]

% Lysis

E:T=2 (corrected value)

| | % Lysis |
|---|---|
| Formulation F | 92.5% |
| Formulation F + succinic acid (pH5.9) | 93.3% |
| Formulation F + succinic acid (pH5.4) | 89.7% |
| Formulation F + succinic acid (pH4.9) | 50.1% |
| Plasma-Lyte A | 87.1% |

E:T=1 (corrected value)

| | % Lysis |
|---|---|
| Formulation F | 72.9% |
| Formulation F + succinic acid (pH5.9) | 74.8% |
| Formulation F + succinic acid (pH5.4) | 69.2% |
| Formulation F + succinic acid (pH4.9) | 29.1% |
| Plasma-Lyte A | 64.4% |

[Fig.7]

**Relative % Lysis**

| pH= | 8.0 | 7.1 | 6.3 | 5.9 | 5.4 | 4.9 |
|---|---|---|---|---|---|---|
| | 1.13 | 1.12 | 1.14 | 1.16 | 1.07 | 0.45 |
| n= | 2 | 1 | 1 | 2 | 1 | 1 |

**Relative 7-AAD-(%)**

| pH= | 8.0 | 7.1 | 6.3 | 5.9 | 5.4 | 4.9 |
|---|---|---|---|---|---|---|
| | 0.97 | 1.02 | 1.11 | 1.36 | 1.33 | 1.13 |
| n= | 2 | 1 | 1 | 2 | 1 | 1 |

31

## INTERNATIONAL SEARCH REPORT

| International application No. |
|---|
| **PCT/JP2024/009978** |

**A.    CLASSIFICATION OF SUBJECT MATTER**

*C12N 1/04*(2006.01)i; *A61K 9/107*(2006.01)i; *A61K 35/17*(2015.01)i; *A61K 47/12*(2006.01)i; *A61K 47/20*(2006.01)i; *A61K 47/26*(2006.01)i; *A61K 47/42*(2017.01)i; *A61P 35/00*(2006.01)i; *C12N 5/0783*(2010.01)i
FI:  C12N1/04; C12N5/0783; A61K47/26; A61K47/12; A61K47/20; A61K47/42; A61K35/17; A61P35/00; A61K9/107

According to International Patent Classification (IPC) or to both national classification and IPC

**B.    FIELDS SEARCHED**

Minimum documentation searched (classification system followed by classification symbols)

C12N1/04; A61K9/107; A61K35/17; A61K47/12; A61K47/20; A61K47/26; A61K47/42; A61P35/00; C12N5/0783

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| X | WO 2021/177279 A1 (GAIA BIOMEDICINE INC.) 10 September 2021 (2021-09-10) claims, examples, paragraphs [0066]-[0082], [0062] | 1, 4-11 |
| Y | claims, examples, paragraphs [0066]-[0082], [0062] | 1–11 |
| Y | JP 2021-502414 A (BIONTECH US INC.) 28 January 2021 (2021-01-28) paragraph [0118] | 1, 4-11 |
| Y | CN 110476950 A (NORTHWEST A&F UNIVERSITY) 22 November 2019 (2019-11-22) claims, examples | 1, 2, 4-11 |
| Y | CN 113201484 A (TIANJIN BOYALEAP TECHNOLOGY CO., LTD.) 03 August 2021 (2021-08-03) claims, examples | 3, 5-11 |
| P, X | WO 2023/038037 A1 (GAIA BIOMEDICINE INC.) 16 March 2023 (2023-03-16) claims, examples | 1-11 |

☐ Further documents are listed in the continuation of Box C.    ☑ See patent family annex.

| | |
|---|---|
| \* | Special categories of cited documents: |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **24 April 2024** | **14 May 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**
Information on patent family members

International application No.

**PCT/JP2024/009978**

| Patent document cited in search report | | | Publication date (day/month/year) | Patent family member(s) | | | Publication date (day/month/year) |
|---|---|---|---|---|---|---|---|
| WO | 2021/177279 | A1 | 10 September 2021 | US 2023/0109717 A1 claims, examples, paragraphs [0112]-[0140], [0108] EP 4151717 A1 | | | |
| JP | 2021-502414 | A | 28 January 2021 | US 2020/0165567 A1 paragraph [0188] WO 2019/094642 A1 | | | |
| CN | 110476950 | A | 22 November 2019 | (Family: none) | | | |
| CN | 113201484 | A | 03 August 2021 | (Family: none) | | | |
| WO | 2023/038037 | A1 | 16 March 2023 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006230396 A
- JP 2002233356 A
- WO 2011021618 A
- WO 2013115322 A
- JP 4947948 B
- WO 2002001952 A
- JP 2013233102 A
- WO 2021177279 A
- JP 2022033487 W
- JP 2018193303 A
- JP 2019170176 A

**Non-patent literature cited in the description**

- **LIU E et al.** *N Engl J Med.*, 2020, vol. 382, 545-53
- **LEONG JW et al.** *Biol. Blood Marrow Transplant*, 2014, vol. 20, 463-473